# EUROPEAN PATENT APPLICATION

(11) **EP 1 959 252 A1**
(43) Date of publication of application: **20.08.2008**
(21) Application number: 08445009.7
(22) Date of filing: 18.02.2008
(51) Int. Cl.: G01N 27/12, G01N 33/00, G08B 17/117

(54) **Gas arrestor system**

(30) Priority: 16.02.2007 US 675996
(71) Applicant: Dometic Corporation, Elkhart, Indiana 46515-0490 (US)
(72) Inventor: McConnell, Patrick N., Goshen Indiana 46528-9072 (US); Buc, Elizabeth C., Roseville Michigan 48066-3942 (US); Lindhagen, Carl H., 591 50 Motala (SE); Karlsson, Arne, 591 72 Motala (SE); Reite, Fredrik, 58212 Linköping (SE)
(74) Representative: Wennborg, Johan

(57) **Abstract**

The subject application relates to a gas arrestor system(s) and/or methodology that facilitate sensing and shutting down a gas-burning appliance via a circuit that can perform both functions. In particular, the system and method can detect specific gases and at specific concentration levels by way of a sensory-power circuit. The circuit performs vapor detection and provides power to various ignition-related portions of the appliance. When gas or gas vapors are detected in the ambient air of the circuit and satisfy a threshold for that particular type of vapor, the circuit is shorted which results in a power loss to at least the ignition-related portions of the appliance. As a result, undesirable ignition of such vapors is mitigated.

## Description

### TECHNICAL FIELD

The subject application generally relates to vapor sensing systems and in particular to a combined vapor-sensor and shutdown control that mitigates undesirable ignition of vapors.

### BACKGROUND

Flammable Vapor Ignition Resistant (FVIR) systems commonly found in water heaters have become a safety standard in the water heater market. When employed, FVIR systems significantly reduce the potential for ignition of flammable vapors outside of the water heater by the water heater's pilot flame. A conventional FVIR system is at least a two-component system that first detects the presence of a vapor using a first component and that secondly stops operation of the particular device using a separate, second component. For example, when flammable vapors are detected by way of one or more sensors, the FVIR system can trigger a circuit or fuse connected thereto to short or open electrically in order to shut down the ignition switch or pilot flame and gas supply to ultimately prevent ignition of the vapors. A variety of flammable vapors such as from everyday cleaning solvents, gasoline, and paint thinners that can be ignited near an open flame or pilot flame; however, sensors typically used in FVIR systems are indiscriminate to the type of vapor they can detect. This can be problematic in some instances such as when the ambient air in proximity of the sensors includes certain vapors originating or produced from other operating conditions that do not necessarily present an ignition risk. Therefore, the predominance of FVIR systems are somewhat customized for water heaters and cannot automatically be utilized by other gas-burning appliances.

### SUMMARY

The following presents a simplified summary in order to provide a basic understanding of some aspects of the systems and/or methods discussed herein. This summary is not an extensive overview of the systems and/or methods discussed herein. It is not intended to identify key/critical elements or to delineate the scope of such systems and/or methods. Its sole purpose is to present some concepts in a simplified form as a prelude to the more detailed description that is presented later.

The subject application relates to a gas arrestor system(s) and/or methodology that facilitate sensing and shutting down a gas-burning appliance via a circuit, wherein a sensor circuit can perform both sensing and shut-down functions nearly simultaneously. According to one aspect of the application, the gas arrestor system comprises at least two sensing resistors arranged in parallel within a power circuit, wherein the at least two sensing resistors are heated and adsorb oxygen on their surfaces to yield a build up of resistance and an isolation barrier between the resistors during normal operation of the gas burning device, and wherein the at least two sensing resistors detect at least one kind of gas at a specific concentration level by way of their voltage output; and a decision component that measures the voltage output from the at least two sensing resistors to determine whether the voltage output correlates to a calibrated level below a lower flammable limit of at least one specific gas at a specific concentration, characterized in that when the voltage output indicates the presence of at least one specified gas at a prescribed concentration, the at least two sensing resistors are shorted, thereby terminating power from the power circuit.

According to another aspect of the application, a gas-arresting method is provided to mitigate undesirable ignition of gas or vapor near or escaping from a gas burning device. The method comprises arranging two sensing resistors in parallel in a power circuit, the power circuit providing power to at least a portion of the gas burning device; measuring a voltage output from the two sensing resistors; and shorting the two sensing resistors when the voltage output indicates that a specified gas is present in ambient air at a specified concentration which results in a termination of power from the power circuit, thereby mitigating a potential fire hazard.

To the accomplishment of the foregoing and related ends, certain illustrative aspects of the invention are described herein in connection with the following description and the annexed drawings. These aspects are indicative, however, of but a few of the various ways in which the principles of the invention may be employed and the subject invention is intended to include all such aspects and their equivalents. Other advantages and novel features of the invention may become apparent from the following detailed description of the invention when considered in conjunction with the drawings.

### BRIEF DESCRIPTION OF THE DRAWINGS

The invention may take physical form in certain parts and arrangement of parts, an example embodiment of which will be described in detail in this specification and illustrated in the accompanying drawings that form a part of the specification. The foregoing and other features and advantages of the present invention will become apparent to those skilled in the art to which the present invention relates upon reading the following description with reference to the accompanying drawings. A brief description of each figure is as follows:

Fig. 1 is a general block diagram of a gas arrestor system that can sense a specific concentration level of a specific gas and shut-down at least an ignition source via the same control.

Fig. 2 is a block diagram of a gas arrestor system that can sense a specific concentration level of a specific gas and shut-down at least an ignition source via the same control when the concentration level or threshold has been satisfied and that can also notify a user of the shut-down.

Fig. 3 is a schematic diagram demonstrating a control in use under normal ambient air conditions.

Fig. 4 is a schematic diagram demonstrating the control of Fig. 3 in use under abnormal ambient air conditions (e.g., one or more prescribed gas levels exceeds threshold).

Fig. 5 is a diagram of an exemplary circuit employed in the gas arrestor system of Figs. 1 and/or 2.

Fig. 6 is a flow diagram of an exemplary method to sense and shutdown at least an ignition source using the same control.

Fig. 7 is a flow diagram of an exemplary method to detect and determine an increasing concentration of at least one gas and to take remedial action to mitigate a potential fire or undesirable ignition risk.

### DETAILED DESCRIPTION

The subject systems and/or methods are now described with reference to the drawings, wherein like reference numerals are used to refer to like elements throughout. In the following description, for purposes of explanation, numerous specific details are set forth in order to provide a thorough understanding of the systems and/or methods. It may be evident, however, that the subject systems and/or methods may be practiced without these specific details. In other instances, well-known structures and devices are shown in block diagram form in order to facilitate describing them.

The subject application relates to a gas arrestor system(s) and/or methodology that facilitate sensing and shutting down a gas-burning appliance via a circuit that can perform both functions. In particular, the gas arrestor system and methodology can be employed in absorption refrigeration units which can operate in the presence of other gases or vapors in the ambient air. Typically, absorption refrigerators are used on recreational vehicles (RVs) and marine vessels or wherever electricity is less reliable, costly, or unavailable. An absorption refrigerator utilizes a heat source to provide the energy that it needs to operate the cooling system. When installed on an RV, for instance, the absorption refrigerator may commonly be exposed to outside air which can include carbon monoxide and/or other exhaust fumes related to the RV idling or traveling on a roadway or dispensing gasoline during re-fueling operations. The outside air can also include vapors from propane being delivered to the appliance (or to another appliance) or from LP gas or propane containers. Conventional FVIR sensor and alarm systems are not discriminative among different gases and thus alarm when any gas or vapor is detected at the prescribed concentration level. Therefore, such FVIR systems cannot be utilized in the dynamic environment of absorption refrigeration units.

Contrary to the FVIR systems, the gas arrestor system as described herein can detect specific types of gases at even lower and more specific concentration levels. In particular, the gas arrestor system involves a sensor-shut-down control in which all or a part of the refrigeration unit can be disabled by virtue of a shorted sensor. Conventional FVIR and other vapor detection systems typically require at least two separate components in order to detect and mitigate a potential fire hazard: (1) a sensor to detect the vapor and (2) a microprocessor to take appropriate action in response to the sensor and/or (3) a separate fuse that is shorted or blown in response to the sensor and/or microprocessor. Unlike such FVIR and other traditional vapor detection systems, the subject system employs a single component to perform both vapor detection and shutdown functions. When the requisite amount or concentration of vapor is detected, potential ignition source(s) associated with the operation of the appliance can be terminated or closed to stop the flow of flammable liquids or gases (e.g., fuel supply) and/or the entire refrigeration unit can be disabled, which would also result in the termination of any ignition sources.

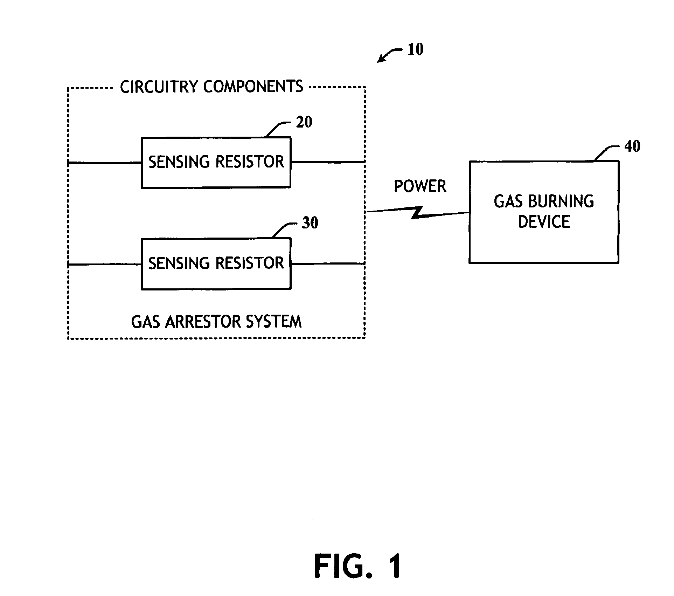

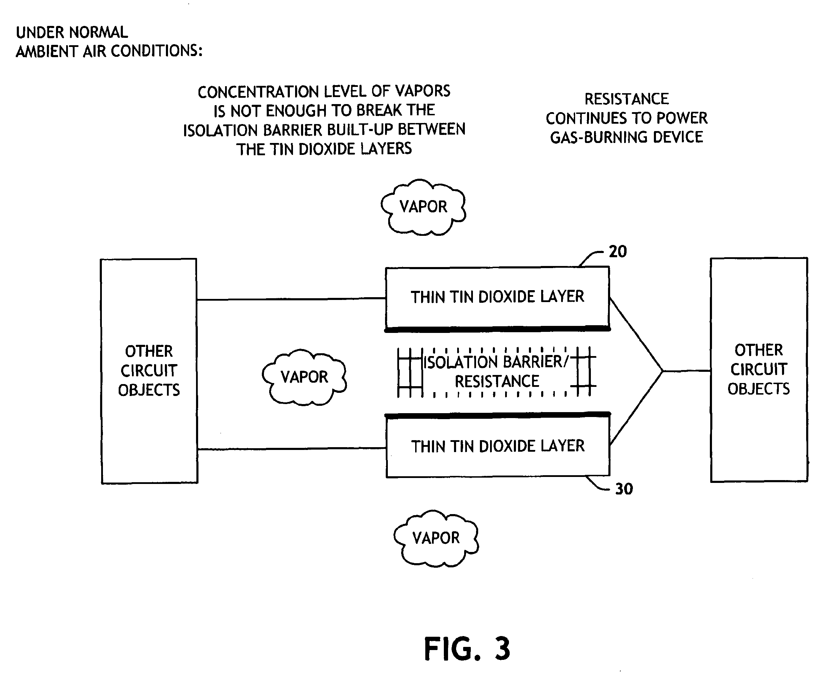

Referring now to Fig. 1, there is illustrated a general block diagram of a gas arrestor system 10 that can sense a specific concentration level of a specific gas and shut-down at least an ignition source via the same control. The system 10 includes at least two thin layers of tin dioxide which serve as first and second sensing resistors 20, 30. The tin dioxide layers are heated and adsorb oxygen on their surfaces as indicated in the figure. This produces a build up of resistance and a creation of an isolation barrier between the tin-dioxide crystals (see Fig. 3, *infra*)*.* If a specific gas is detected at a specific ppm (parts per million) that correlates to a calibrated level below a lower explosion limit, the barrier and resistance are broken, thereby creating a short between the layers or resistors. This short automatically terminates one or more ignition sources of the gas-burning device 40. As a result, the system 10 protects against and mitigates the undesirable ignition of specific flammable vapors and at gas-specific concentrations. The types of gases or vapors include, but are not limited to, ammonia, carbon monoxide, benzene, toluene, LP gas, hydrocarbon-based vapors, and/or any other flammable vapors such as vapors from paint, paint thinner, and solvents.

In practice, for example, imagine that the gas arrestor system 10 is installed in an absorption refrigerator which typically employs ammonia in order to function and cool perishable goods properly. A build-up of ammonia vapors near the pilot light or ignition control may lead to an undesirable ignition of the vapors. With the system 10 in place, the build-up of ammonia vapors near or around the sensing resistors affects the voltage output from the resistors, thus causing a break down in the isolation barrier and resistance; and the resistors are effectively shorted out. Because the power for the one or more ignition sources is controlled at least in part by circuitry components connected to the first and second resistors, a short in the resistors terminates the power to any connected ignition sources.

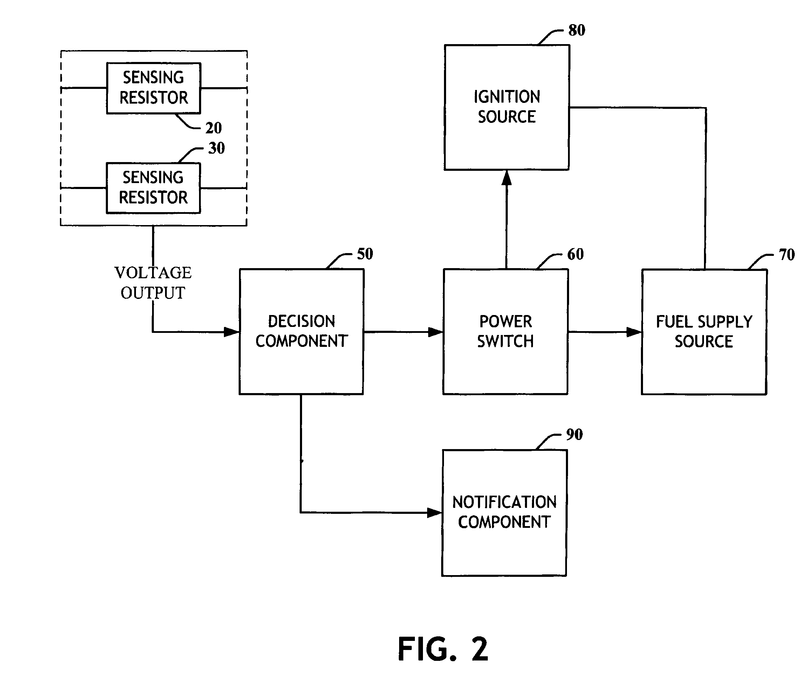

Fig. 2 is a block diagram illustrating additional aspects of the gas arrestor system 10 in Fig. 1. In particular, the first and second sensing resistors 20, 30 can be connected to a decision component 50 such as a microcontroller. Other examples for controlling include but are not limited to threshold sensing devices and opto-isolators.

The decision component 50 can be used to measure a voltage from the specific gas detection sensing resistor (20, 30). Depending on the voltage output of the sensor, the decision component 50 determines whether there are specific gases present at specified concentrations to air or vent and either shuts the system down or allows the system to continue to operate. For instance, when gases are present above the concentration levels for which the sensing resistors are calibrated, the decision component 50 can signal a relay to open the power circuit (switch 60) of the absorption refrigerant system, including any or all fuel supply sources 70 or ignition sources 80. For additional safety concerns, the decision component 50 can include a non-volatile memory that memorizes any occurrence of an arrestor system-based shut down of power and prohibit restarting the refrigerant system without service. Therefore, a user of the refrigerant system is not permitted to manually turn the unit back on before obtaining professional maintenance for the unit. To alert the user of a shutdown, a notification component 90 can be included in the system 10. Notification can involve an audible alarm and/or a visual indicator to make the user aware of the shutdown.

Turning now to Fig. 3, there is illustrated a schematic diagram demonstrating the resistor portion of the gas arrestor system 10 in use under normal ambient air conditions. For example, imagine that the sensing resistors 20, 30 are calibrated to function at an ammonia concentration level at or below 700 ppm. When the ambient air around and passing through the sensing resistors is at or below the desired concentration level of ammonia, an isolation barrier and resistance can build up and be maintained between the sensing resistors, allowing the resistors to output a desirable voltage and any other components connected thereto can function normally. In other words, the desired amount of voltage from the resistors is maintained to power the absorption refrigeration unit to cool and otherwise operate properly.

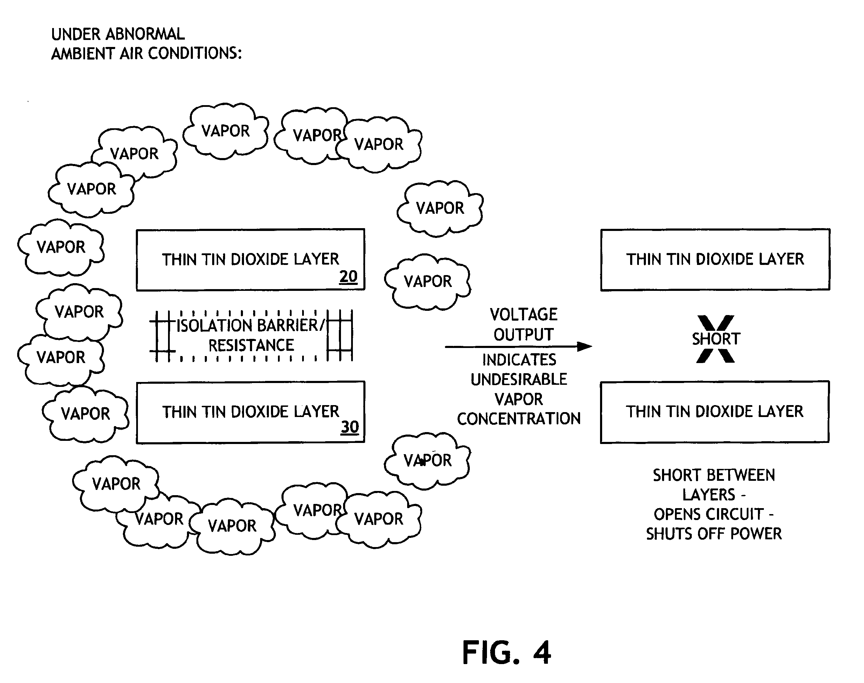

However, as shown in Fig. 4, under abnormal ambient air conditions (e.g., a prescribed gas level exceeds a specific threshold, such as 950 ppm for ammonia), the accumulation of the ammonia gas in the ambient air adversely impacts the voltage output of the resistors. The abnormal voltage output results in a break down of the isolation barrier as well as the resistance built-up between the sensing resistors. Consequently, a short is created between the resistors, causing a drop in voltage and a loss of power for the ignition source, fuel supply, and/or the entire refrigeration unit.

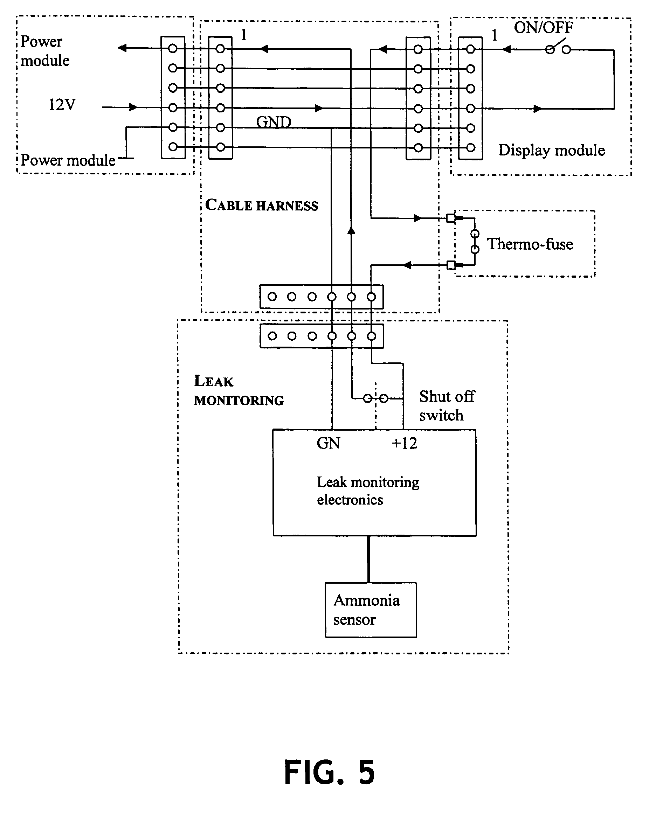

Fig. 5 is a diagram of an exemplary circuit schematic employed in the gas arrestor system of Figs. 1 and/or 2. The configuration of the circuit demonstrates the manner in which the sensing resistors control the power to run the refrigeration system to thereby improve the detection of unintentional vapors near an ignition point (e.g., an LP burner). As mentioned above, the gas arrestor system can be calibrated for specific types of gases and for specific concentration levels that are much lower than conventional vapor detection systems. As a result, the gas arrestor system permits normal operation of gas-burning devices in the presence of some gases while not in the presence of others. This mitigates the occurrence of nuisance tripping which can be highly problematic and troublesome when dealing with food and medicine spoilage concerns.

Though not pictured in the figures, other types of sensing devices can be incorporated into the gas arrestor system 10. For example, a temperature or heat sensor can be included to monitor the temperature near an ignition point or within a particular chamber of a gas-burning device. By doing so, unexpected increases in the temperature can signal an alarm that the device may be malfunctioning such as overheating or not cooling properly or the higher temperature may indicate the presence of a fire or undesirable flames.

Optical sensors can also be employed as a further or entirely independent measure to mitigate a potential fire or the unintentional ignition of flammable vapors. For instance, optical sensors can be used to optically detect a presence of flames or the presence of an ignition that is abnormal to the operation of a burner or other ignition source. Optical sensors can monitor for the presence of flames in chambers or areas of the gas-burning device where flames are not normally found and then shutdown the fuel source or device and alert the user accordingly.

Various methodologies will now be described via a series of acts. It is to be understood and appreciated that the subject system and/or methodology is not limited by the order of acts, as some acts may, in accordance with the subject application, occur in different orders and/or concurrently with other acts from that shown and described herein. For example, those skilled in the art will understand and appreciate that a methodology could alternatively be represented as a series of interrelated states or events, such as in a state diagram. Moreover, not all illustrated acts may be required to implement a methodology in accordance with the subject application.

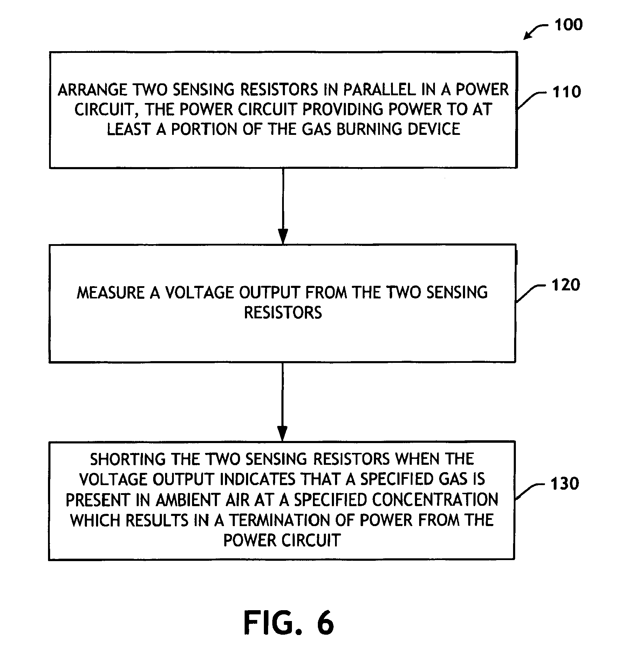

Fig. 6 is a flow diagram of an exemplary method 100 to sense and shutdown at least an ignition source using the same control. The method 100 includes arranging two sensing resistors in parallel in a power circuit, the power circuit providing power to at least a portion of the gas burning device, at 110. In general, the operation of the sensing resistors controls at least one of the following: flow of fuel to an ignition point, the ignition point, and/or power supplied to the gas-burning device. The sensing resistors yield a specific voltage according to the concentration of particular gases present in the air. In the case of an absorption refrigerator, the refrigerator uses ammonia to function and cool foodstuffs. Therefore, the presence of ammonia vapors in the ambient air may be acceptable and not pose a danger at 400 ppm, for example. Under this assumption, the voltage output of the sensing resistors is also suitable to maintain operation of the gas-burning device.

During such routine operation of the device, the sensing resistors are heated and adsorb oxygen on their surface, thereby building up an isolation barrier and resistance between the layers. This resistance effectively completes (or closes) the circuit in order to deliver a sufficient amount of voltage to the fuel supply line or valve, to the ignition point, and/or to the power switch of the device. The voltage output from the sensing resistors can be measured periodically at 120. When the voltage output indicates that a specified gas is present in ambient air at a specified concentration, the method 100 at 130 shorts the two sensing resistors, which effectively terminates power to at least the ignition point from the power circuit. For example, imagine that a voltage output of R corresponds to an ammonia concentration of 900 ppm, which may be below a lower explosion limit but may exceed a desirable ammonia concentration for a particular gas-burning appliance. The voltage output of R can be sufficient to break down the isolation barrier and resistance between the sensing resistors. This drop or loss of voltage opens the circuit, and the appliance turns off almost, if not, immediately thereafter to mitigate an unintentional lighting of the ammonia vapors. Alternatively, the ignition point, burner, or fuel source lines can be closed or deactivated.

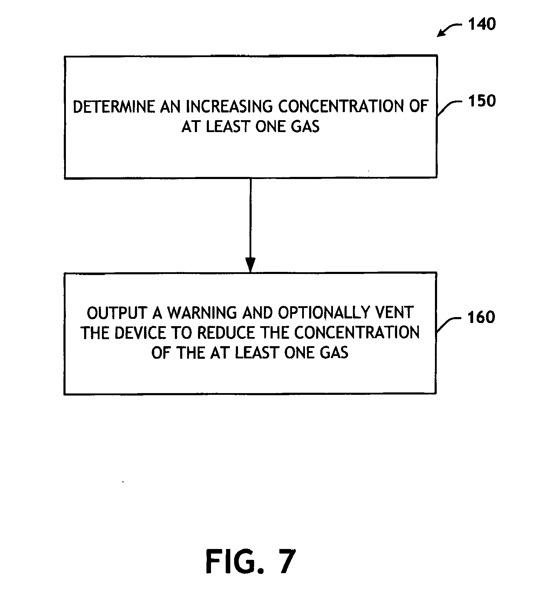

Fig. 7 is a flow diagram of an exemplary method 130 to detect and determine an increasing concentration of at least one gas and to take remedial action to mitigate a potential fire or undesirable ignition risk. The method 140, at 150, includes or involves determining that there is an increasing concentration of at least one gas. This determination can be made in part by examining historical behavior of the device and historical concentration levels of the at least one gas in order to ascertain that the current increase is abnormal or unexpected or undesirable. Upon such determination, the method 140 can output a warning at 160 to notify the user of the current circumstances. Optionally, the device can be automatically vented as triggered by the warning in order to reduce the concentration and mitigate a potentially dangerous situation. Other remedial actions can also be performed upon such determination, such as closing the source of the gas until the concentration of gas decreases (when the source is from within the device). In some cases, the source of the gas may be external to the device, and therefore uncontrollable by the device. When this occurs, the device may continue to operate until the concentration reaches a specific threshold for that gas in order to mitigate a premature shutdown of the device. However, a warning can still be provided with an optional message that the gas vapors are coming from an external source.

What has been described above includes examples of the subject system and/or method. It is, of course, not possible to describe every conceivable combination of components or methodologies for purposes of describing the subject system and/or method, but one of ordinary skill in the art may recognize that many further combinations and permutations of the subject system and/or method are possible. Accordingly, the subject system and/or method are intended to embrace all such alterations, modifications, and variations that fall within the spirit and scope of the appended claims. Furthermore, to the extent that the term "includes" is used in either the detailed description or the claims, such term is intended to be inclusive in a manner similar to the term "comprising" as "comprising" is interpreted when employed as a transitional word in a claim.

## Claims

1. A gas-arrestor system that facilitates termination of an ignitable portion of a gas burning device comprising:
at least two sensing resistors arranged in parallel within a power circuit, wherein the at least two sensing resistors are heated and adsorb oxygen on their surfaces to yield a build up of resistance and an isolation barrier between the resistors during normal operation of the gas burning device, and wherein the at least two sensing resistors detect at least one kind of gas at a specific concentration level by way of their voltage output; and
a decision component that measures the voltage output from the at least two sensing resistors to determine whether the voltage output correlates to a calibrated level below a lower explosion limit of at least one specific gas at a specific concentration,
**characterized in that** when the voltage output indicates the presence of at least one specified gas at a prescribed concentration, the at least two sensing resistors are shorted, thereby terminating power from the power circuit.

2. The system of claim 1, wherein each of the at least two sensing resistors comprise a thin layer of tin dioxide.

3. The system of claim 1, wherein the at least two resistors are exposed to ambient air and output voltage based on content of the ambient air.

4. The system of claim 1, wherein the decision component comprises a non-volatile memory that memorizes an occurrence of a sensing resistor-based power loss and prohibits a restart of at least a portion of the gas burning device before a service reset is performed on the device.

5. The system of claim 1, wherein the power circuit provides power to at least one portion of the gas burning device such that when power from the power circuit is terminated, the at least one portion no longer presents a fire hazard.

6. The system of claim 1, wherein the decision component signals a relay to open the power circuit in ambient air above the concentration level for which the sensing resistors are calibrated.

7. The system of claim 1, wherein the decision component signals activation of a fan to vent air surrounding the gas arrestor system when one or more gases are present at a concentration level that is approaching a shutdown threshold level.

8. The system of claim 1, wherein the at least two sensing resistors are calibrated to detect one or more kinds of gases at prescribed concentration levels by way of their voltage output such that a measured voltage output that is outside of a desired range indicates that a particular gas is present at a concentration level sufficient to alter the voltage output of the resistors.

9. The system of claim 1, further comprising a notification component that alerts a user when power from the power circuit is terminated.

10. The system of claim 9, wherein the notification component comprises at least one of an audible alarm and a visual indicator.

11. The system of claim 9, wherein the notification component is connected to a communication system that sends at least one of a voice or text message to alert the user when the power is terminated.

12. The system of claim 1, wherein the gas burning device is an absorption refrigerant appliance.

13. The system of claim 1, wherein the at least one kind of gas comprises at least one of ammonia, carbon monoxide, benzene, toluene, LP gas, and other flammable vapors comprising vapors from paint and solvents.

14. The system of claim 1, wherein the power circuit provides power to at least one of an ignition source and a fuel supply source.

15. The system of claim 1, wherein the sensing resistors are calibrated on a gas-by-gas basis and according to designated concentration levels for each gas so that the power circuit operates in the presence of some gases but not in the presence of others.

16. A gas-arresting method to mitigate undesirable ignition of gas vapor in a gas burning device comprising:
arranging two sensing resistors in parallel in a power circuit, the power circuit providing power to at least a portion of the gas burning device;
measuring a voltage output from the two sensing resistors; and
shorting the two sensing resistors when the voltage output indicates that a specified gas is present in ambient air at a specified concentration which results in a termination of power from the power circuit, thereby mitigating a potential fire hazard.

17. The method of claim 16, further comprising venting the gas burning device to further mitigate the potential fire hazard upon determining that the voltage output indicates that a specified gas is present at a specified concentration.

18. The method of claim 16, further comprising prohibiting a restart of at least a portion of the gas-burning device following a sensing resistor-based power loss before a service reset is performed on the device.

19. The method of claim 16, further comprising calibrating each of the two sensing resistors to operate and output a desired voltage when designated gas vapors are present in the ambient air in order to facilitate complete operation of the power circuit in the presence of acceptable gas vapors.
